(19) Europäisches Patentamt / European Patent Office / Office européen des brevets

(11) **EP 2 871 974 B1**

(12) **FASCICULE DE BREVET EUROPEEN**

(45) Date de publication et mention de la délivrance du brevet:
**17.08.2016 Bulletin 2016/33**

(21) Numéro de dépôt: **13744719.9**

(22) Date de dépôt: **09.07.2013**

(51) Int Cl.:
**A23L 5/10** (2016.01)

(86) Numéro de dépôt international:
**PCT/FR2013/051629**

(87) Numéro de publication internationale:
**WO 2014/009645 (16.01.2014 Gazette 2014/03)**

(54) **PROCÉDÉ DE CUISSON D' UN ALIMENT ET D'ÉVALUATION DE LA PRÉSERVATION DE SES PROPRIÉTÉS NUTRITIONNELLES**

VERFAHREN ZUM GAREN EINES LEBENSMITTELS UND ZUR BEURTEILUNG DER AUFRECHTERHALTUNG VON DESSEN ERNÄHRUNGSPHYSIOLOGISCHEN EIGENSCHAFTEN

METHOD FOR COOKING A FOOD AND FOR EVALUATING THE PRESERVATION OF THE NUTRITIONAL PROPERTIES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2012 FR 1256831**

(43) Date de publication de la demande:
**20.05.2015 Bulletin 2015/21**

(73) Titulaire: **SEB S.A.**
**69130 Ecully (FR)**

(72) Inventeurs:
• **CUILLERY, Pascal**
**F-74210 Faverges (FR)**

• **HOFLEITNER, Céline**
**F-74150 Rumilly (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A2- 0 032 296**   **US-A- 3 663 232**
**US-A- 4 009 390**   **US-A1- 2011 281 005**

**Description**

L'invention concerne la cuisson d'aliments et l'évaluation de la préservation de leurs propriétés nutritionnelles à l'issue de cette cuisson.

Est concerné le développement d'un procédé de cuisson a priori par contact qui préserve efficacement les qualités nutritionnelles des aliments tout en assurant une réussite culinaire et en limitant, ou facilitant, l'intervention de l'utilisateur. Une référence par rapport à une cuisson standard est notamment prévue.

Il s'agit ici en particulier de proposer une sorte de protocole d'essai qui permette d'apprécier quelle préservation quantifiée des nutriments a pu être atteinte et/ou qu'un aliment a priori moins sec qu'avec une cuisson standard a en outre pu être obtenu.

Cela concerne notamment la cuisson des aliments dans une poêle à induction, et notamment la viande et le poisson.

Il est ici précisé qu'on doit comprendre par « cuisson standard », une cuisson réalisée :

- avec une énergie (telle une puissance) de chauffe assurée par un dispositif de chauffage qui est globalement sensiblement constante dans le temps,
- et/ou avec une température de la surface chauffante (chauffée par un dispositif de chauffage et via laquelle l'aliment cuit) qui augmente globalement dans le temps, tant que la seconde puissance est fournie.

Concernant la « cuisson régulée », on doit la comprendre comme:

- débutant habituellement à température ambiante (de l'ordre de 20°C), avec éventuellement une préchauffe à vide de la surface chauffante (montée en température de cette surface puis stabilisation autour d'une valeur de consigne),
- se poursuivant avec l'ajout de l'aliment au contact de la surface chauffante (baisse alors en général de la température de ladite surface, puis remontée et stabilisation autour d'une seconde valeur de consigne), retournement de l'aliment (baisse alors en général à nouveau de la température de ladite surface puis remontée et stabilisation autour de la - d'une éventuelle troisième
- valeur de cosigne).

Quant à la « cuisson standard », on doit la comprendre:

- comme précédemment, début typique à température ambiante, préchauffe à vide (montée en température, puissance relativement constante), ajout de l'aliment (légère baisse ou stabilisation de la température puis ré-augmentation de la température, puissance relativement constante), retournement de l'aliment (légère baisse ou stabilisation de la température puis augmentation de la température, puissance de nouveau relativement constante).

Ci-après les cuissons respectivement régulée, suivant a1) et/ou a2), et standard, suivant b1) et/ou b2), en fournissent deux références.

A partir de FR-A-2527916, on connait un procédé de cuisson mettant en oeuvre un appareil de cuisson à induction et un article culinaire dont le fond est muni d'un insert ferromagnétique à température de point de Curie déterminé.

Toutefois, ce document ne divulgue pas un procédé mettant en oeuvre un article culinaire présentant un fond comportant une zone ferromagnétique adaptée pour faire de préférence découpler la table à induction une fois la température de Curie atteinte, dans le cadre de la gestion de protocole prévue.

Ainsi, n'y est divulgué aucun protocole d'essai visant à apprécier une préservation des nutriments et/ou une cuisson moins sèche qu'une cuisson standard.

L'invention a notamment pour objectif de pallier tout ou partie des inconvénients de l'art antérieur.

En particulier, un objectif est de définir différentes étapes d'un protocole ou méthode permettant de dégager des intérêts nutritionnels d'une cuisson régulée.

Cela permettra notamment de soutenir le bien-fondé d'allégations nutritionnelles et/ou organoleptiques susceptibles d'être présentées par exemple sur un emballage d'article culinaire ou dans des documents promotionnels.

Un sous-objectif est de dégager les intérêts nutritionnels par rapport à une cuisson standard.

Tout ou partie de ces objectifs est atteint en prévoyant un procédé de cuisson d'un aliment et d'évaluation de la préservation de ses propriétés nutritionnelles, caractérisé en ce qu'il comprend les étapes suivantes :

- fournir au moins deux échantillons crus de l'aliment,
- prédéfinir pour le premier de ces échantillons au moins un temps de cuisson et un protocole de cuisson incluant, pendant ce temps, une cuisson régulée en énergie et/ou en température au contact d'une surface chauffante chauffée par un dispositif de chauffage,

- cuire le premier échantillon selon ledit protocole de cuisson pendant ledit temps de cuisson prédéfini, puis,
- réaliser :

    * un dosage de nutriments contenus dans le premier échantillon cuit, et
    * un dosage de nutriments contenus dans le second échantillon, et

- comparer les dosages entre eux.

Pour renforcer la pertinence des résultats des protocoles de cuisson et d'évaluation évoqués, on recommande par ailleurs que :

- lors de ladite étape de prédéfinition de temps de cuisson, on prédéfinit, pour les échantillons, des temps de cuisson pour différentes évolution d'énergie de chauffe fournies par un dispositif de chauffage et/ou différentes évolutions de température de la surface chauffante,
- lors de ladite étape de conduite de protocole, on conduit deux protocoles différents de cuisson, respectivement pour l'un et l'autre des échantillons, au contact de la surface chauffante, l'un des protocoles incluant une cuisson régulée en énergie et/ou en température, l'autre une cuisson non-régulée, chaque protocole étant réalisé pendant ledit temps prédéfini qui lui correspond, et,

- lors de ladite étape de dosage de nutriments, on réalise :

    * un dosage de nutriments contenus dans les deux échantillons cuits selon les protocoles de cuisson respectifs et/ou
    * un dosage de nutriments contenus dans le premier échantillon cuit selon la cuisson régulée et dans l'aliment cru initial.

Et même de préférence, pour encore davantage de précision et/ou fiabilité des résultats atteints, on conseille que :

- lors de l'étape de prédéfinition des temps de cuisson, on prédéfinisse un premier et un second temps de cuisson de deux échantillons identiques crus de l'aliment, respectivement :

    * pour une première puissance de chauffe régulée ou pour une première évolution de température régulée de chauffe et correspondant respectivement à :

        a1) une puissance de chauffe assurée par le dispositif de chauffage, d'abord sensiblement constante puis qui décroit globalement, après une éventuelle augmentation, diminution et stabilisation suite au contact avec l'échantillon,
        a2) une température de la surface chauffante qui d'abord augmente globalement puis se stabilise à une valeur sensiblement constante, après une éventuelle diminution, augmentation et stabilisation suite au contact avec l'échantillon,

    * pour une seconde puissance de chauffe standard ou pour une seconde évolution de température standard de chauffe et correspondant respectivement à :

        b1) une puissance de chauffe assurée par le dispositif de chauffage qui est globalement sensiblement constante dans le temps,
        b2) une température de la surface chauffante qui augmente globalement dans le temps, tant que la seconde puissance est fournie,

- lors de l'étape de conduite des protocoles de cuisson, on cuise les deux échantillons, respectivement :

    * avec la première puissance de chauffe régulée ou la première évolution de température régulée de chauffe, pendant le premier temps de cuisson,
    * et avec la seconde puissance de chauffe standard ou la seconde évolution de température standard de chauffe, pendant le second temps de cuisson,

- lors de l'étape de dosage, on dose des nutriments contenus dans chacun desdits échantillons cuits et on dose les mêmes nutriments contenus dans l'aliment cru initial.

A priori, et de préférence :

- la comparaison évoquée entre les dosages sera conduite en se rapportant à l'échantillon cru, et/ou
- lors des cuissons, toutes les autres conditions de cuisson non spécifiées ci-avant en référence aux puissances/températures/temps de cuisson seront identiques entre les cuissons ; ainsi, et par exemple, on conseille de retourner chaque échantillon à mi-cuisson (mi-durée de cuisson).

Par ailleurs, on appelle ici :

- temps de cuisson, le temps de contact de l'échantillon (aliment) avec la surface chauffée (plaque pour griller, teppanyaki, poêle...), afin de le cuire,
- énergie délivrée, l'énergie fournie par le dispositif de chauffe, qu'il s'agisse d'une puissance, d'une intensité, d'une tension électrique (ainsi dans le texte, les termes « énergie » et « puissance » sont synonymes, sauf quand une unité SI ou une courbe d'évolution est mentionnée),
- cuisson à coeur, la cuisson réalisée de l'échantillon (aliment) sensiblement au milieu de celui-ci,
- article culinaire : un ustensile de cuisine dans ou sur lequel on cuit par contact un aliment (plaque pour griller, teppanyaki, poêle...), ou encore un accessoire de cuisine, tel un couvercle ou une spatule.

Un problème peut se poser concernant la manière d'assurer notamment une cuisson régulée performante, en particulier dans le cadre du protocole ici évoqué.

A cette fin, il est proposé:

- que, pour cuire, on pourvoit un article culinaire présentant ladite surface chauffante d'un moyen de détection sensible à la température et interrompant une partie au moins de la transmission d'énergie du dispositif de chauffage vers l'article lorsque la température détectée dépasse une valeur déterminée, et/ou
- qu'on pourvoit un article culinaire ou ladite surface chauffante (laquelle appartient soit à l'article culinaire soit au dispositif de chauffage) d'un capteur de mesure physique parmi une température, un flux de chaleur et un débit de vapeur, puis que, lors de ladite étape de cuisson selon le(s) protocole(s), on enregistre la mesure physique, et qu'on renvoie des données enregistrées vers une électronique de traitement qui régulera automatiquement l'énergie de chauffe et/ou qui informera un utilisateur de la nécessité de réguler la cuisson, et/ou,
- que, pour cuire, on place chaque échantillon dans et au contact d'une poêle pourvue intérieurement d'un revêtement antiadhésif, sans ajout de matière grasse.

Un problème peut se poser concernant encore le type de cuisson à assurer, pour conduire de façon pertinente et performante le protocole ici évoqué.

A cette fin, on conseille de cuire les échantillons par contact de ces échantillons avec la surface chauffante, à pression ambiante (dans ce cas, donc hors cuisson vapeur, suivant un principe de cocotte-minute, et hors four).

Un problème peut aussi se poser dans la manière de s'assurer si les niveaux de cuisson prévus ont été atteints.

A cette fin, on conseille de se référer à une température à coeur, ou à un autre paramètre physique de l'aliment.

Plus précisément, on propose ainsi en premier lieu :

- de prédéfinir une (1a) température à coeur prévue de chaque échantillon pour la cuisson à coeur attendue,
- de relever la température à coeur des échantillons, en fin ou après cuisson, en plaçant une ou plusieurs sondes de températures dans chaque échantillon, et
- de comparer la température relevée à celle prédéfinie.

Dans ce cas, pour une performance des mesures, on recommande :

- d'utiliser comme surface chauffante au moins une plaque pour griller ou une poêle (cuisson par contact donc), de placer à son contact l'échantillon à plat, et de disposer l'une au moins des sondes de températures de manière qu'elle s'étende à mi-hauteur de l'échantillon, parallèlement à une surface de l'échantillon en contact avec la plaque pour griller ou la poêle,
- et/ou, si la taille des échantillons est relativement importante (typiquement faux-filet de boeuf ou darne de saumon de 1,5 cm d'épaisseur), de placer plusieurs sondes de températures dans chaque échantillon, de calculer une moyenne des températures relevées et d'utiliser cette moyenne est pour la comparaison précitée.

En alternative à cette température à coeur, il, est proposé :

- avant cuisson, de prédéfinir un marqueur de cuisson parmi la texture et la couleur de l'échantillon considéré, la modification d'une molécule ou macromolécule prévue dudit échantillon pour une cuisson finale prévue,
- de mesurer le marqueur obtenu en fin ou après cuisson,
- et de comparer les mesures réalisées à celle prédéfinie.

Pour une performance du traitement des résultats/relevés obtenus, on recommande par ailleurs que :

- lors de ladite comparaison entre les valeurs mesurées et celles prédéfinies, on vérifie que l'écart entre elles est inférieur ou égal à 5%, à 40% près, selon le test de Student, et/ou,
- qu'après le dosage des échantillons, une analyse statistique de Student soit conduite, avec un test de Student appairé si les échantillons à comparer sont issus d'un même poisson, et/ou,
- qu'avant et après la cuisson, chaque échantillon soit pesé pour calculer une perte en poids de l'échantillon causée par la cuisson (si elle est faible, cette valeur est révélatrice d'une performance de la cuisson en termes de préservation des nutriments et de cuisson peu asséchante).

Un autre problème peut se poser relativement au traitement des échantillons après cuisson, afin de préserver la pertinence des mesures à effectuer.

A cette fin, il est proposé :

- qu'après la cuisson et avant le dosage, les échantillons soient amenés à une température négative, en °C, et/ou
- qu'avant le dosage les échantillons soient broyés.

Encore un autre problème peut se poser relativement à la manière de conduire les cuissons régulée et/ou standard afin qu'en particulier dans le premier cas, les résultats obtenus soient les plus performants possibles en termes de préservation des nutriments et/ou de cuisson peu asséchante.

A cette fin, il est proposé :

- que, pour la cuisson à première puissance de chauffe régulée ou première évolution de température régulée et à 10% près au maximum, on aboutisse à une température de la surface chauffante d'environ 200°C, et/ou la puissance de chauffe délivrée est d'abord inférieure à 1800W, pour terminer à moins de 800W, et/ou
- que, pour la cuisson à seconde puissance de chauffe standard ou seconde évolution de température standard de et à 10% près au maximum, on délivre une puissance qui est globalement entre 1750W et 2250W, et/ou on atteigne une température de la surface chauffante d'environ 250°C à l'issue dudit second temps de cuisson.

D'autres caractéristiques et/ou avantages pourront ressortir de la description complémentaire ci-après, fournie à titre non limitatif, en référence aux dessins annexés, dans lesquels :

- la figure 1 schématise des article culinaire et accessoire de cuisine, avec une boucle de régulation, avec un chauffage par induction;
- la figure 2 est une vue partielle en coupe verticale d'une poêle adaptée à un chauffage par induction ;
- les figures 3,4 schématisent respectivement un chauffage par une source de gaz et une source électrique ;
- la figure 5 montre de dessous un article culinaire adapté à un chauffage par indiction ;
- la figure 6 montre des évolutions d'énergie/température

en fonction de durées prédéterminées dans le cadre d'un ou plusieurs protocoles de chauffage/cuisson eux-mêmes prédéfinis ;

- les figures 7,8 montrent une implantation de sondes de température dans un échantillon (la figure 8 est une vue suivant la flèche VIII de la figure 7);
- la figure 9 schématise le montage de thermocouples utilisables comme moyens de détection ;
- et la figure 10 présente les profils température au centre de la surface chauffante 10, ainsi que la puissance (en W) mesurée au niveau de la plaque de cuisson en fonction du temps.

Le protocole concerné par le mode de réalisation présenté ci-après est basé sur la comparaison de deux modes de cuisson poêlés :

- une cuisson non-régulée, dite standard dans l'exemple (poêle 1 de diamètre 26 cm, obtenue par frappe à froid, chauffage de la poêle sur un foyer à induction 3 de diamètre 21cm, plaque « ThorTI63R ®»). La puissance fournie par la plaque lorsque la poêle est remplie d'eau est en moyenne de 1855W (3 essais), soit un débit d'évaporation de 0,67 gramme d'eau par seconde (3 essais),

- une cuisson régulée à environ 205°C (poêle 2 de diamètre 26 cm, obtenue par frappe à froid avec trois bandes de matériau Phytherm® 200, chauffage de la poêle sur un foyer à induction 3 de 21cm avec plaque ThorTI63R ®). La puissance fournie par la plaque lorsque la poêle est remplie d'eau est en moyenne de 1445W (3 essais), soit un débit d'évaporation de 0,49 gramme d'eau par seconde (3 essais).

On aurait pu évaluer la préservation des propriétés nutritionnelles en ne comparant qu'une cuisson régulée avec les propriétés nutritionnelles de l'aliment cru.

Il suffira alors, avant cuisson, de prédéfinir pour au moins un premier des échantillons à tester au moins un temps de cuisson et le protocole de cuisson associé, régulée en énergie et/ou en température au contact de la surface chauffante 10 chauffée par le dispositif de chauffage 3.

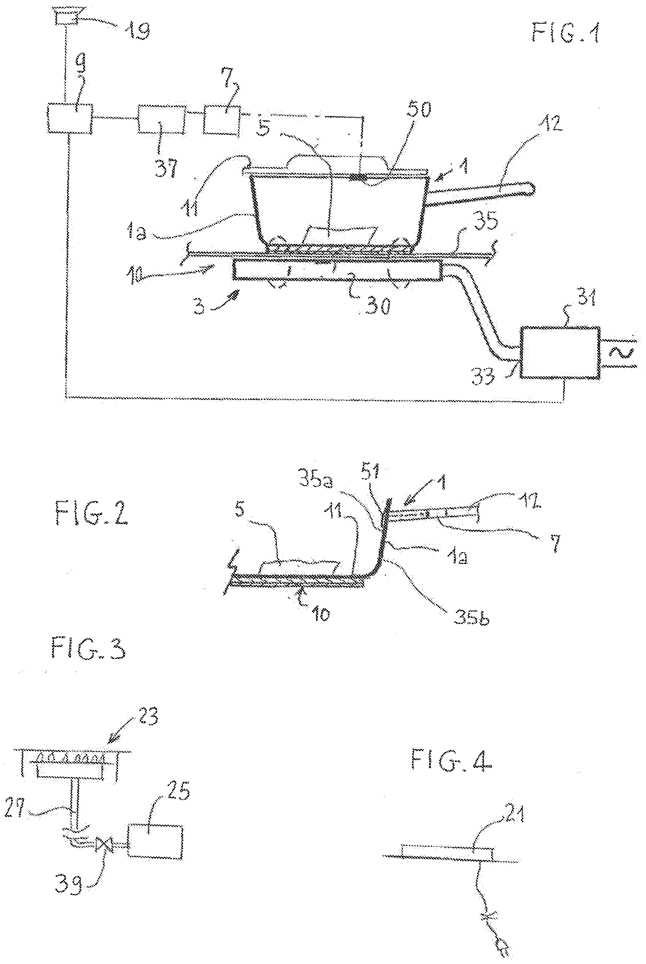

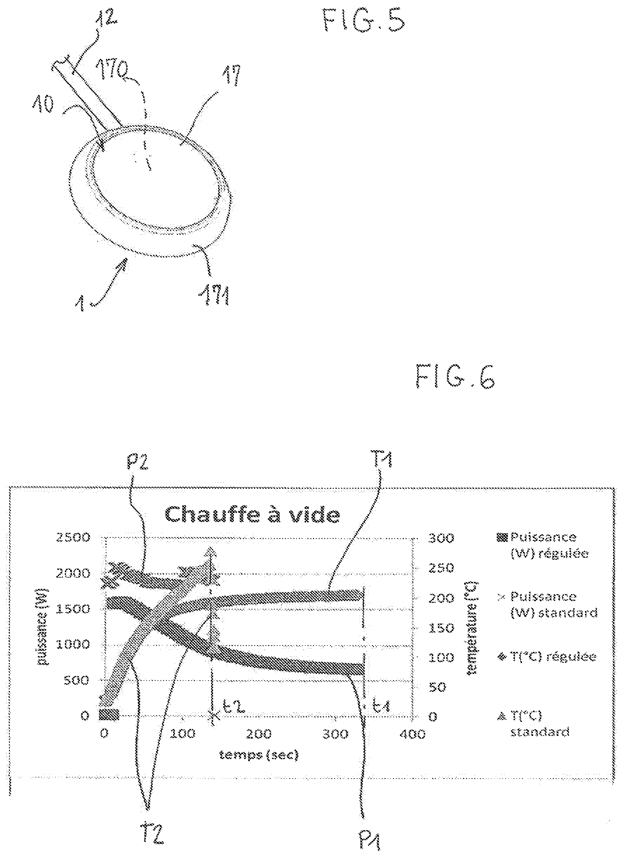

Dans l'exemple, la surface chauffante 10 est le fond de la poêle ou casserole 1 (voir figure 1 ou 5), suivant la cuisson concernée.

En alternative, ce pourrait être par exemple la paroi latérale de la poêle, ou une plaque grill à induction sur laquelle serait directement cuit l'aliment concerné.

Ainsi, on cuira le(s) échantillon(s) 5 par contact de ces échantillons avec la surface chauffante 10, à pression ambiante.

On a ici choisi, pour cuire, de placer chaque échantillon dans et au contact d'une poêle 1 intérieurement pourvue d'un revêtement antiadhésif 11, en PTFE par exemple, sans ajout de matière grasse. Et cette cuisson qui s'opère par contact avec la surface chauffante est à pression ambiante (atmosphérique).

Avant d'appliquer le protocole retenu, on va donc dans l'exemple fournir ici au moins deux échantillons crus de l'aliment, en l'espèce d'origine animal.

A cet égard, il convient de noter que la perte en vitamines d'un aliment au cours de la cuisson peut typiquement dépendre de paramètres comme :

- la composition de la matière première (taux de lipides, protéines, glucides...)
- la teneur initiale en nutriments (aliment cru),
- le morceau de l'animal choisi et de la préparation des échantillons,
- le mode de cuisson,
- le niveau de cuisson (plus ou grillé ou cuit à coeur...).

Dans l'exemple, on a sélectionné une viande et/ou un poisson.

Pour la viande, on a retenu des tranches (ici de faux-filet) provenant d'un même animal (ou si besoin de nombreuses tranches d'animaux de même race, même âge). On donc obtenu des tranches pratiquement identiques : Les faux-filets ont été raidis (-2°C) puis tranchés de manière à obtenir des tranches de 1,5 cm d'épaisseur. Les échantillons été numérotés en fonction de leur ordre (de la tête à la queue) et en fonction de s'ils proviennent du morceau droit ou gauche.

Pour le poisson, le saumon a été choisi. Les poissons provenait d'un même élevage et étaient quasi du même calibre (taille, poids). Les saumons ont été tranchés à la machine de la tête à la queue pour obtenir des darnes de 1,5 cm d'épaisseur. Les darnes ont été numérotées en commençant par les darnes côté tête. Seules les 12 darnes du milieu ont été gardées (darnes 3 à 14) de manière à minimiser la variation du taux lipidique intra individu. Les darnes ont été coupées en deux pour former des demi-darnes. L'arête centrale et la peau ont été enlevées.

De manière à maîtriser la variabilité intra-individu (tête-queue, droite-gauche), les demi-darnes de chaque saumon ont été différenciées en fonction de la droite et la gauche du saumon puis réparties successivement entre la cuisson standard, la cuisson régulée et le cru. Pour les différents saumons, la première demi-darne (darne numéro 3, droite) correspond successivement à une cuisson standard, une cuisson régulée ou un échantillon cru comme illustré dans le tableau suivant :

|  | Saumon 1 | Saumon 2 | Saumon 3 | Saumon 4 |
|---|---|---|---|---|
| Darne 3 Droite (D3D) | Cuisson standard | Cuisson régulée | Echantillon cru | Cuisson standard |
| Darne 3 Gauche (D3G) | Cuisson régulée | Echantillon cru | Cuisson standard | Cuisson régulée |
| 4D | Echantillon cru | Cuisson standard | Cuisson régulée | Echantillon cru |

(suite)

|  | Saumon 1 | Saumon 2 | Saumon 3 | Saumon 4 |
|---|---|---|---|---|
| D4G | Cuisson standard | Cuisson régulée | Echantillon cru | Cuisson standard |
| D5D | Cuisson régulée | Echantillon cru | Cuisson standard | Cuisson régulée |
| D5G | Echantillon cru | Cuisson standard | Cuisson régulée | Echantillon cru |
| D6D | Cuisson standard | Cuisson régulée | Echantillon cru | Cuisson standard |
| ... | ... | ... | ... | ... |
| D14G | Echantillon cru | Cuisson standard | Cuisson régulée | Echantillon cru |

Avant la cuisson les échantillons sont légèrement épongés à l'aide d'un papier absorbant et sont pesés.

Par ailleurs, avant cuisson selon le protocole concerné, on a donc prédéfini (voir figure 6) un premier t1 et un second t2 temps de cuisson des deux échantillons identiques crus de l'aliment, respectivement :

- pour une première puissance de chauffe régulée P1 figure 2, ou pour une première évolution de température régulée de chauffe T1, et correspondant, comme on le voit, respectivement à :

    * une puissance de chauffe assurée par le dispositif de chauffage 3, d'abord sensiblement constante puis qui décroît globalement, après une éventuelle augmentation, diminution et stabilisation suite au contact avec l'échantillon 5 (évolutions non représentées puisque les courbes sont « à vide »),
    * une température de la surface chauffante 10 qui d'abord augmente globalement puis se stabilise à une valeur sensiblement constante, après une éventuelle diminution, augmentation et stabilisation (à nouveau non représentées) suite au contact avec l'échantillon,

- pour une seconde puissance de chauffe standard P2, ou pour une seconde évolution de température standard T2 de chauffe et correspondant respectivement à :

    * une puissance de chauffe assurée par le dispositif de chauffage 3 qui est globalement sensiblement constante dans le temps,
    * une température de la surface chauffante 10 qui augmente globalement dans le temps, tant que la seconde puissance est fournie.

Toujours avant la cuisson, on a encore pourvu l'article culinaire 1, ici sa surface chauffante 10 (mais avec une cuisson directement sur une plaque à griller cela aurait pu être la plaque elle-même), d'un capteur de mesure physique 5 parmi une température, un flux de chaleur et un débit de vapeur. Grâce à cela on va pouvoir, lors de l'étape de cuisson selon le(s) protocole(s), enregistrer sur l'enregistreur 7 (figure 1) la mesure physique et renvoyer des données enregistrées vers l'électronique de traitement 9 qui régulera automatiquement l'énergie de chauffe et/ou qui informera (via une alerte 19, tel un afficheur ou un message sonore) un utilisateur de la nécessité de réguler la cuisson, selon le protocole en cause.

De fait, on a donc équipé un accessoire d'article culinaire, ici le couvercle 11 de la poêle 1, d'un moyen de détection 50 sensible à un paramètre physico-chimique et interrompant, via par exemple l'électronique de traitement 9 et dans le cadre du protocole, une partie au moins de la transmission d'énergie du dispositif de chauffage 3 vers l'article 1, lorsque le paramètre détecté dépasse une valeur déterminée.

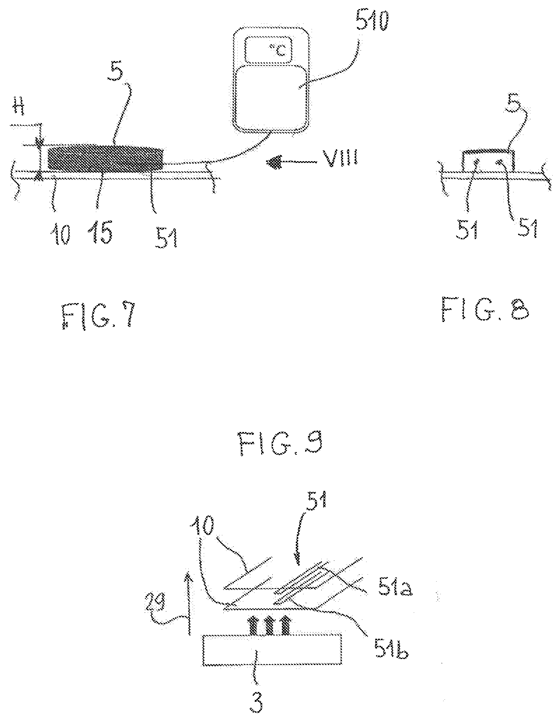

Dans l'exemple, pour déterminer un niveau de cuisson et pouvoir comparer les deux modes de cuisson, en s'assurant que l'on est arrivé au même niveau de cuisson, on a choisi de mesurer la température à coeur des échantillons 5 après cuisson grâce à au moins une sonde thermocouple 51 relié au lecteur de mesures 510 ; voir figure 7. Comme montré figure 8, la sonde a été insérée parallèlement à la surface 15 de l'échantillon en contact avec la surface de cuisson 10.

La surface chauffante utilisée étant la poêle 1 (avec l'alternative de la plaque à griller), on va placer à son contact l'échantillon 5 à plat, et disposer l'une au moins des sondes 51 de manière qu'elle s'étende à mi-hauteur H/2 de l'échantillon, parallèlement à la surface 15 de l'échantillon en contact avec la poêle (respectivement la plaque pour griller) ; voir figure 7.

Compte tenu de l'épaisseur des échantillons et pour sécuriser les mesures, on a placé plusieurs sondes de températures 51 dans chaque échantillon 5 puis calculé une moyenne des températures relevées, cette moyenne ayant finalement été utilisée pour la comparaison finale.

En complément, ou plutôt en substitution de ces sondes de températures 51, on aurait pu, avant cuisson :

- prédéfinir au moins un marqueur de cuisson parmi :

  * la texture (tel : moelleux, croustillant, dureté, croquant, tendreté,...) et la couleur de l'échantillon considéré, ceci étant apprécié au toucher ou visuellement par un opérateur, et
  * via une analyse (physico-)chimique, la modification d'une molécule ou macromolécule (telle : dénaturation, gélatinisation, hydrolyse) prévue de l'échantillon pour la cuisson finale prévue,

- mesurer (ou apprécier) le marqueur obtenu en fin ou après cuisson,
- puis comparer les mesures réalisées à celle prédéfinie. Plus précisément, on aurait pu ainsi pu conduire :

  * une analyse sensorielle avec un panel d'experts (descripteurs prédéfinis),
  * une analyse de dénaturation des protéines de l'échantillon (DSC : Differential Scanning Calorimetry

    - détection par calorimétrie différentielle, fluorimétrie,...),

  * une analyse de la texture (texturomètre : compression, pénétration, dureté, traction, force à la rupture,...), par exemple un texturomètre TA XT2 ou TA plus une analyse de la couleur (réflectance, systèmes L*a*b,...).

Concernant l'article culinaire 1, s'il s'agit d'une poêle ou plus généralement d'un récipient à poignée(s) de levage, on recommande a priori d'utiliser un article compatible induction à surface chauffante 10, et une source inductive associée comme dispositif de chauffage 3.

Il sera alors aisé, pour l'étape de cuisson régulée, que la surface 10 soit automatiquement régulée, en particulier à une température de $205 \pm 10°C$.

Pour cela, on recommande, comme illustré figure 5, que l'article culinaire comporte un fond pourvu d'une plaque (un disque dans l'exemple) 17 en acier inoxydable (ferro) magnétique. Ce fond peut présenter une épaisseur d'1 mm environ et être soudé à la face inférieure d'une plaque de fond 170 plus épaisse en aluminium ou cuivre qui peut être elle-même soudée au fond 171 de la carcasse en acier inoxydable formant la calotte creuse de la poêle illustrée.

La surface de la plaque 17 pourra être déterminée de façon à ne plus (ou moins) être détectée par l'inducteur 30 de la table à induction 3 et être ainsi découplée, une fois la température de Curie atteinte (dans le cas précité environ 205°C).

Si, pour déterminer un niveau de cuisson et/ou pouvoir comparer les deux modes de cuisson précités, on a retenu la solution de la/des sondes de températures 51 dans l'échantillon, on conseille que le matériau magnétique de la partie 17 ait un point de Curie égal (à +/- 2°C) à ladite température prédéterminée à laquelle le découplage précité avec l'inducteur 30 doit intervenir et que la/chaque sonde 51 doit détecter comme température de seuil. On a retenu dans l'exemple une température entre 45 et 75°C (correspond à la plage de cuisson de bleu à bien cuit), avec un choix en fonction de la cuisson souhaitée (voir tableau ci-après).

Bien évidemment, on pourrait prévoir que le matériau magnétique de la partie 17 ait un point de Curie égal, à +/- 15°C près, à ladite température prédéterminée à laquelle le découplage précitée avec l'inducteur 30 doit intervenir.

Dans l'hypothèse d'une comparaison des deux modes de cuisson, régulée et standard comme précitées, et du choix de la cuisson (donc de la température de fin de cuisson) à coeur comme marqueur de cuisson prédéfini, il est nécessaire de s'assurer que l'on arrive au même niveau de cuisson pour les deux mêmes échantillons cuits selon lesdits protocoles respectifs. Pour cela, on recommande comme indiqué ci-avant que la/les sondes thermiques 50 s'étend(nt) à mi-hauteur H/2, la température variant notablement, suivant le placement de la sonde dans l'échantillon. Pour le saumon et le faux-filet, on retiendra de préférence trois sondes espacées d'environ 1 à 2 cm et la moyenne de ces mesures qui sera enregistrée.

Dans les essais menés, on a cherché pour les cuissons de faux-filets à obtenir une cuisson à point (température moyenne des essais à coeur des échantillons : 63°C).

Les niveaux de cuisson et leurs températures correspondantes sont présentés dans le tableau suivant :

| Degré de cuisson | Température à coeur |
|---|---|
| Bleu | 54 à 35°C |
| Saignant | 56 à 58°C |
| Rosé | 59 °C |

(suite)

| Degré de cuisson | Température à coeur |
|---|---|
| A point | 60 à 62°C |
| Bien cuit juteux | 62 à 68°C |
| Bien cuit sec | + de 68 °C |

Pour le saumon, on a regardé la température à coeur à laquelle la ligne rosée disparaissait (température moyenne des essais à coeur des échantillons : 75°C).

On préférera que les temps de cuisson en fonction des modes de cuisson soient fixés de manière à ne pas présenter de différence significative à 5%, à 40% près, (test de Student) au niveau de la température mesurée à coeur après (en fin de) cuisson.

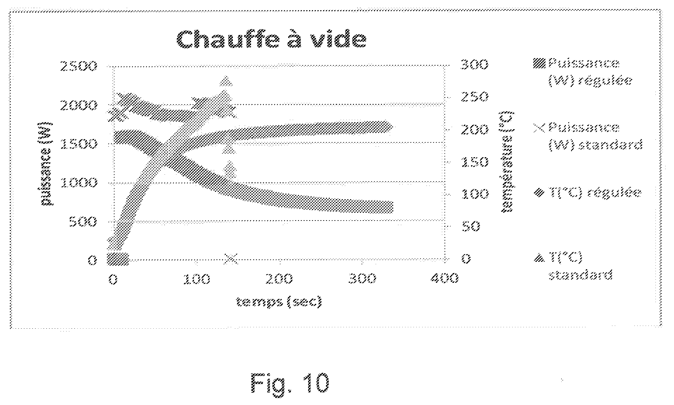

Dans les essais menés, on a conduits les protocoles de cuisson avec des poêles 1 chauffées à la puissance maximale sans matière grasse. Les profils température au centre de la surface chauffante 10, ainsi que la puissance (en W) mesurée au niveau de la plaque de cuisson en fonction du temps sont présentés en figure 10.

En pratique :

- pour la cuisson régulée, le chauffage débute à température ambiante, puis il y a eu : préchauffe à vide (sans article culinaire et/ou sans aliment), montée en température puis stabilisation autour d'une valeur de consigne), ensuite placement de l'aliment sur la surface de cuisson (avec alors baisse de la température puis remontée et stabilisation autour de la valeur de consigne), et retournement de l'aliment (baisse de la température puis remontée et stabilisation autour de la valeur de consigne).

- pour l'autre cuisson : à nouveau début à température ambiante, préchauffe à vide (montée en température, puissance relativement constante), placement de l'aliment sur la surface de cuisson (avec légère baisse ou stabilisation de la température puis augmentation de la température, avec puissance relativement constante) et retournement de l'aliment (légère baisse ou stabilisation de la température puis augmentation de la température, avec encore puissance relativement constante).

Les échantillons (une tranche de faux-filet et 2 demi-darnes de saumon par poêle) ont été placés au centre de la poêle après 65% du temps de préchauffe (préchauffe = temps nécessaire pour atteindre 205°C à +-5°C), soit une fois que celui-ci a atteint 200°C à +-5°C pour la cuisson régulée et 220°C à +-5°C pour la cuisson standard. La puissance (en W) du chauffage n'a pas été modifiée. Les échantillons ont été retournés à la mi-cuisson.

Pour la viande, les échantillons cuits suivant la cuisson standard l'on été 1min45sec (temps t2) par face. Avec la cuisson régulée ils ont été cuits 2min15sec (temps t1) par face.

Pour le saumon les échantillons ont été cuits 2min15sec par face, dans les deux cas (temps t1, t2).

Pour la cuisson régulée et à 10% près au maximum, on a abouti à une température de la surface chauffante d'environ 200°C, et/ou la puissance de chauffe délivrée a d'abord été inférieure à 1800W, pour terminer à moins de 800W.

Pour l'autre cuisson, toujours à 10% près au maximum, on a délivré une puissance qui a été globalement maintenue entre 1750W et 2250W, et/ou la surface chauffante 10 a atteint une température d'environ 250°C à l'issue du temps de cuisson correspondant (dit ci-avant second temps de cuisson.

Après cuisson, les échantillons ont été pesés pour calculer leur perte en poids causée par la cuisson. Ensuite, la température à coeur a été mesurée de la même manière que pour fixer le niveau de cuisson de façon à vérifier que l'on n'était toujours pas significativement différent à 5% (test de Student) et que l'on parvenait toujours au même niveau de cuisson.

Après cuisson, et avant le dosage, les échantillons sont amenés à une température négative, en °C. On conseille de les congeler pour préservation.

Avant dosage, ils sont en outre de préférence broyés, y compris celui cru.

Concernant le traitement des données, la mesure du poids avant (cru) et après cuisson permet de calculer la perte en poids (en %) engendrée par le mode de cuisson. Pour la comparaison entre les modes de cuisson, il peut être important de comparer la perte en nutriments par rapport au cru.

Par exemple, pour le dosage de la vitamine B6 pour la viande, la méthode utilisée pour le dosage a été celle référencée EN14663 accréditée COFRAC. Celle pour le poisson avec dosage de la vitamine B3, la méthode a été utilisée.

Les résultats, présentés en mg de vitamine par 100g de produit ont été rapportés en mg de vitamine par gramme de produit initial (cru) en tenant compte de la perte en poids pour les échantillons cuits d'après la formule suivante :

$$C_{cuit} = C_f \times \left(1 - \frac{\Delta P}{100}\right)$$

Avec Ccuit (mgvit B6/gviande initial) la concentration de vitamines B6 après cuisson par gramme de viande crue, Cf (mgvit B6/gviande initial) la concentration de vitamine B6 après cuisson par gramme de viande cuite, et $\Delta P$ la perte en poids au cours de la cuisson.

La perte en vitamine B6 (notée $\Delta$vit B6) a été calculée de la manière suivante :

$$\Delta vit\ B6 = 100 \times \frac{C_{cru} - C_{cuit}}{C_{cru}}$$

Concernant le traitement statistique, pour la viande, une analyse de Student à 5% est utilisée pour savoir si les deux modes de cuisson sont statistiquement différents ou pas.

Ici dans le cas du poisson, un test de Student appairé a été utilisé. En effet la comparaison entre les deux modes de cuisson s'est faite par échantillon, ici par saumon.

Ainsi, de façon générale, conseille t'on qu'après le dosage des échantillons, une analyse statistique de Student soit conduite, avec un test de Student appairé si les échantillons à comparer sont issus d'un même animal, ici un poisson, et sont quasi identiques entre eux.

Les résultats peuvent alors être exprimés comme une préservation des nutriments (exemple : la cuisson régulée préserve X % en plus de vitamine que la cuisson régulée) ou comme une limitation de la perte des nutriments (exemple : la cuisson régulée permet de perdre X % de moins en vitamine que la cuisson standard).

Les résultats des essais menés ont été les suivants :

| Limitation de la perte en poids | | |
|---|---|---|
| Aliment | Perte en poids cuisson standard (%) ± ET | Perte en poids nouveau procédé de cuisson (%) ± ET |
| Saumon | 22,50 ± 2,88 | 16,88 ± 2,10 |
| Faux-filet essai 1 | 16,91 ± 1,55 | 12,27 ± 1,16 |
| Faux-filet essai 2 | 15,49 ± 0,64 | 12,80 ± 1,28 ou 11,35 ± 0,88 |

| Limitation de la perte en vitamine, et plus particulièrement les vitamines hydrosolubles (B3 et B6). | | |
|---|---|---|
| Aliment (vitamine) | Perte vitamine cuisson standard (%) ± ET | Perte vitamine nouveau procédé de cuisson (%) ± ET |
| Saumon (vitamine B3) | 10,32 ± 5,63 | 6,26 ± 4,52 |
| Faux-filet (vitamine B6) essai 1 | 21,27 ± 4,98 | 8,51 ± 4,12 |
| Faux-filet (vitamine B6) essai 2 | 29,99 ± 8,89 | 17,55 ± 9,01 ou 13,16 ± 11,09 |
| ET : Ecart type | | |

Bilan sur la cuisson régulée:

| Différence par rapport à la cuisson standard | Faux-filet | | Saumon |
|---|---|---|---|
| | 205°C | 215°C | 205°C |
| Perte en poids | OK -17,41% | OK -26,74% | OK -23,44% |
| Perte en vitamine B6 | OK -41,49% | OK -56,11% | / |
| Perte en vitamine B3 | / | / | OK -39,36% |
| OK = significativement différent à 5% | | | |

Parmi les avantages apportés par la solution proposée ci-avant, on relève que le/chaque protocole d'essai permet:

- de mettre en évidence un intérêt nutritionnel d'un mode de cuisson par rapport à un autre. Il est facilement reproductible et fiable ;
- de mesurer la perte en poids (majoritairement perte en eau). Une différence de perte en poids peut être caractérisée par un intérêt nutritionnel ;
- de maitriser la variabilité de la matière première et apporte une analyse statistique pour vérifier la différence (statistiquement différent à 5%, à 40% près) des modes de cuisson.

Le protocole peut aussi être adapté pour mesurer l'impact du mode de cuisson sur la dégradation des huiles de cuisson.

De ce qui précède, on peut conclure que, pour évaluer la préservation des propriétés nutritionnelles d'au moins un aliment, on utilisera au moins deux échantillons crus de l'aliment et, pour le premier (au moins), on prédéfinira au moins un temps de cuisson et un protocole de cuisson régulée. Le second échantillon où le dosage de nutriments sera effectué pourra demeurer cru.

Deux protocoles différents de cuisson, respectivement pour l'un et l'autre des (au moins deux) échantillons, au contact de la surface chauffante permet de comparer cuisson régulée en énergie et/ou en température et cuisson non-régulée. Et leurs particularités sont respectées, puisque chaque protocole est réalisé pendant le temps prédéfini qui lui correspond.

Concernant la surface chauffante 10 (appelée aussi surface de cuisson), elle appartiendra soit au dispositif de chauffage 3 soit à l'article culinaire. Le dispositif 3 pourra assurer un chauffage par induction. Ainsi, il pourra comprendre la bobine ou inducteur 30 (figure 1) alimentée par l'onduleur 31 tirant son énergie du courant électrique fourni par le secteur. L'onduleur 31 fournit sur sa sortie 33 un courant électrique alternatif ayant de préférence une forte intensité et sous faible tension. Au-dessus de cette bobine est disposée une plaque isolante 35, par exemple en verre, sur laquelle peut être posée, au droit de la bobine 1, une casserole ou poêle à fond magnétique 10.

Par exemple si la plaque verre 35 est remplacée par un grill, telle une plaque ondulée électriquement isolante, la surface chauffante 10 appartiendra au dispositif de chauffage 3.

La surface chauffante 10 pourrait aussi être une plaque chauffée par-dessous par :

- une résistance électrique 21 raccordée à une source d'alimentation, telle le secteur, (voir figure 4), ou
- un brûleur à gaz 23 alimenté en gaz comburant (typiquement air) et en gaz combustible par une source 25 par exemple propane, via la conduite 27 (voir figure 3).

On peut aussi imaginer que la surface chauffante 10 soit la paroi latérale la d'un article culinaire, porteuse d'une résistance électrique.

Concernant maintenant le capteur 50 ou 51 de mesure physique, il pourra donc être sensible à une température ou un flux de chaleur ou un débit de vapeur.

Un thermocouple pourra être un capteur de température performant. Il peut être placé sur ou dans l'article culinaire 1 (au niveau de la couche d'aluminium ou de l'inox pour un tel article du type à calotte et poignée(s) : poêle, casserole...), sur la surface de cuisson 10, au niveau de la poignée 12 de l'article ou encore au niveau d'un couvercle (tel 11), comme schématisé figures 1,2 ou 5, ou autre ustensile ou accessoire qui entre en jeu au cours de la cuisson (spatule...).

Comme montré figure 9, deux thermocouples 51a,51b placés successivement, typiquement en superposition, dans le sens du flux de chaleur 29 permettent de détecter une différence de températures à un temps donné et donc de définir le capteur de flux de chaleur 51.

Dans le cas d'un capteur de débit de vapeur, on peut prévoir, comme montré figure 2, que l'article culinaire, tel le haut de la jupe de l'article 1, ou un autre ustensile ou accessoire de cuisine (couvercle, spatule) inclut une structure (telle la jupe la de l'article ou le bloc couvercle 11) présentant une première et une deuxième faces 35a,35b opposées l'une à l'autre, et au moins ce moyen de détection 51, ici sensible aux composés volatils issu de la cuisson. Le moyen de détection 51 est relié au dispositif de traitement 7 apte à émettre un signal s'il y a une détection desdits composés volatils. Par souci de facilité de mise en oeuvre et d'emploi, le moyen de détection peut être intégré au moins à la première face 35a, laquelle sera agencée pour être au contact ou en regard d'un aliment. On recommande en outre que cette première face soit nue ou revêtue d'une couche de revêtement antiadhésif en matériau sol-gel ou en résine fluorocarbonée formant un réseau continu fritté. Quant au moyen de détection 51, on conseille qu'il comprenne :

- un matériau actif apte à modifier ses propriétés de conduction électrique en présence des composés volatils);
- au moins deux éléments de connexion assurant la connexion entre le matériau actif et le dispositif de traitement.

Concernant son fonctionnement, on prévoit favorablement que ce moyen de détection et le dispositif de traitement 7 forment un circuit électrique où le matériau actif présentera une résistivité variant en présence des composés volatils et, si un courant électrique traversant le dispositif de traitement devient différent d'un seuil prédéterminé, le dispositif de traitement émettra un signal.

Une boucle de régulation permettant la cuisson régulée et couplée au dispositif de traitement pour notamment adapter l'énergie électrique délivrée, en fonction des données/signaux émis par le détecteur 51 de composés volatils, est envisagée.

Lors de l'étape de cuisson selon le(s) protocole(s), la mesure physique/chimique/physico-chimique concernée sera enregistrée en mémoire 37, et les données enregistrées pourront être envoyées vers l'électronique de traitement 9 qui pourra:

- réguler automatiquement l'énergie de chauffe (par exemple en modifiant l'énergie délivrée par la résistance électrique 21 ou par l'onduleur 30, ou encore en agissant sur une vanne 39 interposée sur la conduite 27),
- et/ou qui informera l'utilisateur de la nécessité de réguler la cuisson, par exemple par le moyen d'alerte ou d'affichage 19 ; voir figure 1.

## Revendications

1. Procédé de cuisson d'un aliment et d'évaluation de la préservation de ses propriétés nutritionnelles, **caractérisé en ce qu'**il comprend les étapes suivantes :

    - fournir au moins deux échantillons (5) crus de l'aliment,
    - prédéfinir pour le premier de ces échantillons au moins un temps de cuisson et un protocole de cuisson incluant, pendant ce temps, une cuisson régulée en énergie et/ou en température au contact d'une surface chauffante (10) chauffée par un dispositif de chauffage (3),
    - cuire le premier échantillon (5) selon ledit protocole de cuisson, pendant ledit temps de cuisson prédéfini, puis,
    - réaliser :

        * un dosage de nutriments contenus dans le premier échantillon cuit (5), et
        * un dosage de nutriments contenus dans le second (5) échantillon, et

    - comparer les dosages entre eux.

2. Procédé selon la revendication 1, **caractérisé en ce que** :

    - lors de ladite étape de prédéfinition de temps de cuisson, on prédéfinit, pour les échantillons (5), des temps de cuisson pour différentes évolution d'énergie de chauffe fournies par un dispositif de chauffage (3) et/ou différentes évolutions de température de la surface chauffante (10),
    - lors de ladite étape de conduite de protocole, on conduit deux protocoles différents de cuisson, respectivement pour l'un et l'autre des échantillons (5), au contact de la surface chauffante (10), l'un des protocoles incluant une cuisson régulée en énergie et/ou en température, l'autre un cuisson non-régulée, chaque protocole étant réalisé pendant ledit temps prédéfini qui lui correspond, et,
    - lors de ladite étape de dosage de nutriments, on réalise :

        * un dosage de nutriments contenus dans les deux échantillons (5) cuits selon les protocoles de cuisson respectifs et/ou.
        * un dosage de nutriments contenus dans le premier échantillon cuit selon la cuisson régulée et dans l'aliment, cru initial.

3. Procédé selon la revendication 1 ou 2, **caractérisé en ce que** :

    - lors de l'étape de prédéfinition des temps de cuisson, on prédéfinit un premier et un second temps de cuisson des deux échantillons (5) identiques crus de l'aliment, respectivement :

        * pour une première puissance de chauffe régulée ou pour une première évolution de température régulée de chauffe et correspondant respectivement à :

. une puissance de chauffe assurée par le dispositif de chauffage (3), d'abord sensiblement constante puis qui décroit globalement, après une éventuelle augmentation, diminution et stabilisation suite au contact avec l'échantillon (5),

. une température de la surface chauffante (10) qui d'abord augmente globalement puis se stabilise à une valeur sensiblement constante, après une éventuelle diminution, augmentation et stabilisation suite an contact avec l'échantillon,

\* pour une seconde puissance de chauffe standard ou pour une seconde évolution de température standard de chauffe et correspondant respectivement à :

. une puissance de chauffe assurée par le dispositif de chauffage (3) qui est globalement sensiblement constante dans le temps,

. une température de la surface chauffante (10) qui augmente globalement dans le temps, tant que la seconde puissance est fournie, et

- lors de l'étape de conduite des protocoles de cuisson, on cuit les deux échantillons (5), respectivement :

\* avec à première puissance de chauffe régulée ou la première évolution de température régulée due chauffe, pendant le premier temps de cuisson,

\* et avec la seconde puissance de chauffe standard ou la seconde évolution de température standard de chauffe, pendant le second temps de cuisson,

**4.** Procédé selon l'une des revendications 1 à 3, **caractérisé en ce que**, pour cuire, on pourvoit un article culinaire (1) présentant ladite surface chauffante (10) d'un moyen, de détection (50,51) sensible a la température et interrompant une partie au moins de la transmission d'énergie du dispositif de chauffage (3) vers l'article (1) lorsque la température détectée dépasse une valeur déterminée.

**5.** Procédé selon l'une des revendications 1 à 3,
**caractérisé en ce qu'**on pourvoit un article culinaire (1) ou ladite surface chauffante (10), laquelle appartient soit à l'article culinaire soit au dispositif de chauffage (3), d'un capteur (50, 51) de mesure physique parmi une température, un flux de chaleur (29) et un débit de vapeur, lors de ladite étape de cuisson selon le(s) protocole(s) on enregistre la mesure physique, et on renvoie des données enregistrées vers une électronique de traitement (9) qui régule automatiquement l'énergie de chauffe et/ou qui informe un utilisateur de la nécessité de réguler la cuisson.

**6.** Procédé selon l'une des revendications 1 à 5, **caractérisé en ce que** le dispositif de chauffage (3) étant un appareil de chauffage par induction (3), on pourvoit l'article d'un matériau magnétique (17) présentant un point de Curie égal à +/- 15°C à ladite température déterminée.

**7.** Procédé selon l'une des revendications 1 à 6, **caractérisé en ce qu'**on cuit les échantillons (5) par contact de ces échantillons avec la surface chauffante (10), à pression ambiante.

**8.** Procédé selon l'une des revendications 1 à 7, **caractérisé en ce que** :

- avant cuisson, on prédéfinit un marqueur de cuisson parmi la texture et la couleur de l'échantillon (5) considéré, la modification d'une molécule ou macromolécule prévue dudit échantillon pour une cuisson finale prévue,
- on mesure le marqueur obtenu en fin ou après cuisson,
- on compare les mesures réalisées à celle prédéfinie.

**9.** Procédé selon l'une des revendications 1 à 7, **caractérisé en ce que** :

- avant cuisson, on prédéfinit une température à coeur prévue de chaque échantillon (5) pour la cuisson à coeur attendue,
- on mesure la température à coeur des échantillons (5), en fin ou après cuisson, en plaçant une ou plusieurs sondes de températures (51) dans chaque échantillon (5), et
- on compare la température mesurée à celle prédéfinie.

**10.** Procédé selon la revendication 9, **caractérisé en ce qu'**on utilise comme surface chauffante (10) au moins une plaque (17, 170) pour griller ou une poêle (1), on place à son contact l'échantillon (5) à plat, et on dispose l'une au moins des sondes (51) de manière qu'elle s'étende à mi-hauteur de l'échantillon, parallèlement à une surface de l'échantillon (5) en contact avec la plaque (17, 170) pour griller ou la poêle (1).

11. Procédé selon la revendication 9 ou 10, **caractérisé en ce qu'**on place plusieurs sondes de températures (51) dans chaque échantillon (5), on calcule une moyenne des températures relevées et cette moyenne est utilisée pour ladite comparaison.

12. Procédé selon l'une des revendications 8 à 11, **caractérisé en ce que** lors de ladite comparaison entre les valeurs mesurées et celles prédéfinies, on vérifie que l'écart entre elles est inférieur ou égal à 5%, à 40% près, selon le test de Student.

13. Procédé selon l'une des revendications 1 à 12, **caractérisé en ce qu'**avant et après la cuisson, chaque échantillon (5) est pesé pour calculer une perte en poids de l'échantillon causée par la cuisson.

14. Procédé selon l'une des revendications 1 à 13, **caractérisé en ce qu'**après la cuisson et avant le dosage, les échantillons (5) sont amenés à une température négative, en °C.

15. Procédé selon l'une des revendications 1 à 14, **caractérisé en ce qu'**avant le dosage les échantillons (5) sont broyés.

16. Procédé selon l'une des revendications 1 à 15, **caractérisé en ce qu'**après le dosage des échantillons (5), une analyse statistique de Student est conduite, avec un test de Student appairé si les échantillons à comparer sont issus d'un même poisson.

17. Procédé selon l'une des revendications 1 à 16, **caractérisé en ce que**, pour cuire, on place chaque échantillon (5) dans et au contact d'une poêle (1) pourvue intérieurement d'un revêtement antiadhésif (11), sans ajout de matière grasse.

18. Procédé selon l'une des revendications 1 a 17, **caractérisé en ce que**, pour la cuisson régulée et à 10% près au maximum, on aboutit à une température de la surface chauffante (10) d'environ 200°C, et/ou la puissance de chauffe délivrée est d'abord inférieure à 1800W, pour terminer à moins de 800W.

19. Procédé selon la revendication 3, **caractérisé en ce que**, pour la cuisson à seconde puissance de chauffe standard ou seconde évolution de température standard, et à 10% près au maximum, on délivre une puissance qui est globalement entre 1750W et 2250W, et/ou on atteint une température de la surface chauffante (10) d'environ 250°C à l'issue dudit second temps de cuisson.

**Patentansprüche**

1. Verfahren zum Garen eines Lebensmittels und zur Bewertung der Konservierung seiner ernährungsphysiologischen Eigenschaften, **dadurch gekennzeichnet, dass** es die folgenden Schritte umfasst:

- Bereitstellen von mindestens zwei rohen Proben (5) des Lebensmittels,
- Vordefinieren für die erste dieser Proben von mindestens einer Garzeit und einem Garprotokoll, umfassend, während dieser Zeit, ein hinsichtlich Energie und/oder Temperatur geregeltes Garen in Kontakt mit einer Heizfläche (10), geheizt von einer Heizvorrichtung (3),
- Garen der ersten Probe (5) gemäß dem Garprotokoll, während der vordefinierten Garzeit, dann
- Ausführen:

-- einer Dosierung von Lebensmitteln, die in der ersten (5) Probe enthalten sind, und
-- einer Dosierung von Lebensmitteln, die in der zweiten (5) Probe enthalten sind, und

- Vergleichen der Dosierungen untereinander.

2. Verfahren nach Anspruch 1, **dadurch gekennzeichnet, dass**

- während des Schritts des Vordefinierens der Garzeit für die Proben (5) Garzeiten für verschiedene Entwicklungen der Heizenergie, bereitgestellt von einer Heizvorrichtung (3), und/oder verschiedene Entwicklungen der Temperatur der Heizfläche (10) vordefiniert werden,
- während des Schritts des Durchführens des Protokolls zwei verschiedene Garprotokolle jeweils für die eine und für die andere Probe (5), in Kontakt mit der Heizfläche (10), durchgeführt werden, wobei eines der Protokolle ein geregeltes Garen hinsichtlich der Energie und/oder der Temperatur, das andere ein nicht geregeltes Garen umfasst, wobei jedes Protokoll während der vordefinierten Zeit durchgeführt wird, die ihm entspricht, und
- während des Schritts des Dosierens von Lebensmitteln Folgendes ausgeführt wird:

-- eine Dosierung von Lebensmitteln, die in den zwei Proben (5), enthalten sind, und gegart gemäß den entsprechenden Garprotokollen, und/oder
-- eine Dosierung von Lebensmitteln, die in der ersten Probe enthalten sind, gegart gemäß dem geregelten Garen und im anfänglichen rohen Lebensmittel.

3. Verfahren nach Anspruch 1 oder 2, **dadurch gekennzeichnet, dass**:

- während des Schritts des Vordefinierens der Garzeit eine erste und eine zweite Garzeit der zwei identischen rohen Proben (5) des Lebensmittels vordefiniert werden, jeweils:

-- für eine erste geregelte Heizleistung oder für eine erste Entwicklung der geregelten Heiztemperatur und jeweils Folgendem entsprechend:

--- einer Heizleistung, sichergestellt durch die Heizvorrichtung (3), zunächst im Wesentlichen konstant, dann allgemein abnehmend, nach einer eventuellen Erhöhung Verringerung und Stabilisierung nach dem Kontakt mit der Probe (5),
--- eine Temperatur der Heizfläche (10), die sich zunächst allgemein erhöht, dann auf einen im Wesentlichen konstanten Wert stabilisiert, nach einer eventuellen Verringerung, Erhöhung und Stabilisierung nach dem Kontakt mit der Probe,

-- für eine zweite Standard-Heizleistung oder für eine zweite Entwicklung der Standard-Heiztemperatur und jeweils entsprechend dem Folgenden:

--- eine Heizleistung, sichergestellt durch die Heizvorrichtung (3), die allgemein im Wesentlichen konstant während der Zeit ist,
--- eine Temperatur der Heizfläche (10), die sich allgemein während der Zeit erhöht, so dass die zweite Leistung bereitgestellt wird, und

- während des Schritts des Durchführens des Garprotokolls, Garen der zwei Proben (5), jeweils:

-- mit der ersten geregelten Heizleistung oder der ersten Entwicklung der geregelten Heiztemperatur während der ersten Garzeit,
-- und mit der zweiten Standard-Heizleistung oder der zweiten Entwicklung der Standard-Heiztemperatur während der zweiten Garzeit.

4. Verfahren nach einem der Ansprüche 1 bis 3, **dadurch gekennzeichnet, dass** zum Garen ein Kochartikel (1), der die Heizfläche (10) aufweist, mit einem Nachweismittel (50, 51) versehen wird, das empfindlich gegenüber der Temperatur ist und einen Teil mindestens der Übertragung von Energie der Heizvorrichtung (3) hin zum Artikel (1) unterbricht, wenn die nachgewiesene Temperatur einen vorbestimmten Wert übersteigt.

5. Verfahren nach einem der Ansprüche 1 bis 3, **dadurch gekennzeichnet, dass** ein Kochartikel (1) oder die Heizfläche (10), die entweder zum Kochartikel oder zur Heizvorrichtung (3) gehört, mit einem Sensor (50, 51) zur physischen Messung eines einer Temperatur, eines Heizflusses (29) und eines Dampfdurchsatzes versehen wird, während des Schritts des Garens nach dem/den Protokoll(en) die physische Messung registriert wird und die registrierten Daten zu einer elektronischen Verarbeitung (9) geschickt werden, die automatisch die Heizenergie regelt und/oder die einen Benutzer über die Notwendigkeit informiert, das Garen zu regeln.

6. Verfahren nach einem der Ansprüche 1 bis 5, **dadurch gekennzeichnet, dass**, da die Heizvorrichtung (3) eine Induktions-Heizvorrichtung (3) ist, der Artikel mit einem magnetischen Material (17) versehen wird, das einen Curie-Punkt gleich +/- 15 °C bei der vorbestimmten Temperatur aufweist.

7. Verfahren nach einem der Ansprüche 1 bis 6, **dadurch gekennzeichnet, dass** die Proben (5) durch Kontakt dieser Proben mit der Heizfläche (10) bei Umgebungsdruck gegart werden.

8. Verfahren nach einem der Ansprüche 1 bis 7, **dadurch gekennzeichnet, dass**

- vor dem Garen ein Garmarker aus des Textur und der Farbe der berücksichtigten Probe (5), der vorgesehenen Modifizierung eines Moleküls oder Makromoleküls der Probe für ein vorgesehenes endgültiges Garen vordefiniert wird,
- der erhaltene Marker am Ende des und nach dem Garen(s) gemessen wird,
- die durchgeführten Messungen mit der Vordefinierten verglichen werden.

9. Verfahren nach einem der Ansprüche 1 bis 7, **dadurch gekennzeichnet, dass**

- vor dem Garen eine vorgesehene Kerntemperatur jeder Probe (5) für das erwartete Kerngaren vordefiniert wird,
- die Kerntemperatur der Proben (5) am Ende und nach dem Garen gemessen wird, indem eine oder mehrere Temperatursonden (51) in jeder Probe (5) angebracht werden,
- die gemessene Temperatur mit der Vordefinierten verglichen wird.

10. Verfahren nach Anspruch 9, **dadurch gekennzeichnet, dass** als Heizfläche (10) mindestens eine Platte (17, 170) zum Grillen oder eine Pfanne (1) verwendet wird, die Probe (5) damit flach in Kontakt gebracht wird und mindestens eine der Sonden (51) derart angebracht wird, dass sie sich auf halber Höhe der Probe, parallel zu einer Fläche der Probe (5), in Kontakt mit der Platte (17, 170) zum Grillen oder der Pfanne (1) erstreckt.

11. Verfahren nach Anspruch 9 oder 10, **dadurch gekennzeichnet, dass** mehrere Temperatursonden (51) in jeder Probe (5) angebracht werden, ein Durchschnitt der erhobenen Temperaturen berechnet und dieser Durchschnitt für den Vergleich verwendet wird.

12. Verfahren nach einem der Ansprüche 8 bis 11, **dadurch gekennzeichnet, dass** beim Vergleich zwischen den gemessenen und den vordefinierten Werten überprüft wird, dass der Abstand zwischen ihnen geringer oder gleich 5 %, etwa 40 % gemäß dem Student-Test ist.

13. Verfahren nach einem der Ansprüche 1 bis 12, **dadurch gekennzeichnet, dass** vor und nach dem Garen jede Probe (5) gewogen wird, um einen Gewichtsverlust der Probe, hervorgerufen durch das Garen, zu berechnen.

14. Verfahren nach einem der Ansprüche 1 bis 13, **dadurch gekennzeichnet, dass** nach dem Garen und vor dem Dosieren die Proben (5) auf eine negative Temperatur in °C gebracht werden.

15. Verfahren nach einem der Ansprüche 1 bis 14, **dadurch gekennzeichnet, dass** vor dem Dosieren die Proben (5) zerkleinert werden.

16. Verfahren nach einem der Ansprüche 1 bis 15, **dadurch gekennzeichnet, dass** nach dem Dosieren der Proben (5) eine statistische Student-Analyse mit einem gepaarten Student-Test durchgeführt wird, wenn die Proben, die verglichen werden sollen, von einem gleichen Fisch stammen.

17. Verfahren nach einem der Ansprüche 1 bis 16, **dadurch gekennzeichnet, dass**, zum Garen jede Probe (5) in und in Kontakt mit einer Pfanne (1), die im Inneren mit einer Antihaftbeschichtung (11) versehen ist, ohne Zugabe von Fett angebracht wird.

18. Verfahren nach einem der Ansprüche 1 bis 17, **dadurch gekennzeichnet, dass** für das geregelte Garen und bei etwa 10 % vom Maximum eine Temperatur der Heizfläche (10) von ungefähr 200 °C erreicht wird, und/oder die gelieferte Heizleistung zunächst weniger als 1800 Watt beträgt, um bei weniger als 800W zu enden.

19. Verfahren nach Anspruch 3, **dadurch gekennzeichnet, dass** zum Garen bei einer zweiten Standard-Heizleistung oder zweiten Entwicklung der Standardtemperatur und bei etwa 10 % vom Maximum eine Leistung bereitgestellt wird, die allgemein zwischen 1750W und 2250W liegt, und/oder eine Temperatur der Heizfläche (10) von ungefähr 250 °C am Ende der zweiten Garzeit erzielt wird.

**Claims**

1. Method for cooking a food and for evaluating the preservation of the nutritional properties thereof, **characterised in that** it comprises the following steps:

   - providing at least two raw samples (5) of the food,
   - predefining for the first of these samples at least one cooking time and a cooking protocol including, during this time, energy-regulated and/or temperature-regulated cooking in contact with a heating surface (10) heated by means of a heating device (3),
   - cooking the first sample (5) according to said cooking protocol, for said predefined cooking time, then,
   - carrying out:

     -- an assay of nutrients contained in the cooked first sample (5), and
     -- an assay of nutrients contained in the second sample (5), and

   - comparing the assays with one another.

2. Method according to claim 1, **characterised in that**:

   - during said step of predefining the cooking time, for the samples (5), cooking times for various changes in heating energy supplied by a heating device (3) and/or various changes in the temperature of the heating surface (10) are predefined,
   - during said step of protocol conducting, two different cooking protocols are conducted, respectively for one and the other of the samples (5), in contact with the heating surface (10), with one of the protocols including an energy-regulated and/or temperature-regulated cooking, the other a non-regulated cooking, with each protocol being carried out during said predefined time that corresponds to said protocol, and,
   - during said step of assaying nutrients, the following are carried out:

     -- an assay of nutrients contained in the two cooked samples (5) according to the respective cooking protocols and/or
     -- an assay of nutrients contained in the cooked first sample according to the regulated cooking and in the initial raw food.

3. Method according to claim 1 or 2, **characterised in that**:

   - during the step of predefining cooking times, a first and a second cooking time of the two identical raw samples (5) of the food are defined, respectively:

     -- for a first regulated heating power or for a first change in the regulated heating temperature and corresponding respectively to:

       --- a heating power provided by the heating device (3), first substantially constant then which globally decreases, after a possible increase, decrease and stabilisation following contact with the sample (5),
       --- a temperature of the heating surface (10) which first globally increases then stabilises at a substantially constant value, after a possible decrease, increase and stabilisation following contact with the sample,

     -- for a second heating power standard or for a second change in the standard heating temperature and corresponding respectively to:

       --- a heating power provided by the heating device (3) which is substantially globally constant over time,
       --- a temperature of the heating surface (10) which globally increases over time, as long as the second power is provided, and

   - during the step of cooking protocol conducting, the two samples (5) are cooked, respectively:

     -- with the first regulated heating power or the first change in the regulated heating temperature, during the first cooking time,

17

-- and with the second heating power standard or the second change in the standard heating temperature, during the second cooking time.

4. Method according to one of claims 1 to 3, **characterised in that**, for cooking, a cooking utensil (1) having said heating surface (10) is provided with a means of detection (50, 51) sensitive to the temperature and interrupting at least a portion of the transmission of energy from the heating device (3) to the utensil (1) when the temperature detected exceeds a determined value,

5. Method according to one of claims 1 to 3, **characterised in that** a cooking utensil (1) or said heating surface (10), which belongs either to the cooking utensil or to the heating device (3), is provided with a sensor (50, 51) for the measurement of physical feature among a temperature, a flow of heat (29) and a flow rate of steam, during said step of cooking according to the protocol(s) the physical feature measurement is recorded, and the recorded data is sent to processing electronics (9) which automatically regulate the heating energy and/or which inform a user of the need to regulate the cooking.

6. Method according to one of claims 1 to 5, **characterised in that** the heating device (3) being an induction heating device (3), the utensil is provided with a magnetic material (17) that has a Curie point equal to +/- 15°C at said determined temperature.

7. Method according to one of claims 1 to 6, **characterised in that** the samples (5) are cooked via contact of these samples with the heating surface (10), at ambient pressure.

8. Method according to one of claims 1 to 7, **characterised in that**:

   - before cooking, a cooking marker is predefined among the texture and the colour of the sample (5) considered, the modification of a molecule or macromolecule provided of said sample for a final cooking provided,
   - the marker obtained at the end or after cooking is measured,
   - the measurements taken are compared with the predefined measurement.

9. Method according to one of claims 1 to 7, **characterised in that**:

   - before cooking, a core temperature provided for each sample (5) for the expected core cooking is predefined,
   - the core temperature of the samples (5) is measured, at the end or after the cooking, by placing one or several temperature probes (51) in each sample (5), and
   - the measured temperature is compared with the predefined temperature.

10. Method according to claim 9, **characterised in that** at least one grilling plate (17, 170) or a pan (1) is used as a heating surface (10), the sample (5) is placed flat in contact with it, and at least one of the probes (51) is arranged in such a way that it extends at mid-height of the sample, parallel to a surface of the sample (5) in contact with the grilling plate (17, 170) or the pan (1).

11. Method according to claim 9 or 10, **characterised in that** several temperature probes (51) are placed in each sample (5), an average of the temperatures taken is calculated and this average is used for said comparison.

12. Method according to one of claims 8 to 11, **characterised in that** during said comparison between the measured values and the predefined values, it is verified that the difference between them is less than or equal to 5%, within 40%, according to the Student test.

13. Method according to one of claims 1 to 12, **characterised in that** before and after the cooking, each sample (5) is weighed in order to calculate a loss in weight of the sample caused by the cooking.

14. Method according to one of claims 1 to 13, **characterised in that** after the cooking and before the assay, the samples (5) are brought to a negative temperature, in °C.

15. Method according to one of claims 1 to 14, **characterised in that** before the assay the samples (5) are ground.

16. Method according to one of claims 1 to 15, **characterised in that** after the assay of the samples (5), a Student statistical analysis is conducted, with a paired Student test if the samples to be compared come from the same fish.

**17.** Method according to one of claims 1 to 16, **characterised in that**, for cooking, each sample (5) is placed in and in contact with a frying pan (1) provided interiorly with a non-stick coating (11), without adding fat.

**18.** Method according to one of claims 1 to 17, **characterised in that**, for regulated cooking and within a maximum of 10%, a temperature of the heating surface (10) of about 200°C is reached, and/or the heating power delivered is first less than 1800W, for terminating at less than 800W.

**19.** Method according to claim 3, **characterised in that**, for the cooking at a second standard heating power or second change in standard temperature and within a maximum of 10%, a power that that is globally between 1750W and 2250W is delivered, and/or a temperature of the heating surface (10) of about 250°C is reached at the end of said second cooking time.

FIG.1

FIG.2

FIG.3

FIG.4

FIG.5

FIG.6

FIG.7

FIG.8

FIG.9

Fig. 10

**EP 2 871 974 B1**

**RÉFÉRENCES CITÉES DANS LA DESCRIPTION**

**Documents brevets cités dans la description**

- FR 2527916 A